# EUROPEAN PATENT APPLICATION

(11) **EP 4 617 361 A1**
(43) Date of publication of application: **17.09.2025**
(21) Application number: 22965101.3
(22) Date of filing: 08.11.2022
(51) Int. Cl.: C12N 1/21, C12N 15/60, C12P 7/42, C12N 9/88

(54) **4-HYDROXYBENZOIC ACID-PRODUCING TRANSFORMANT OF BACTERIUM BELONGING TO GENUS HYDROGENOPHILUS**

(71) Applicant: Utilization of Carbon Dioxide Institute Co.,Ltd., Tokyo 135-0091 (JP)
(72) Inventor: YUKAWA, Hideaki, Tokyo 135-0091 (JP)
(74) Representative: Vossius & Partner Patentanwälte Rechtsanwälte mbB
(86) International application number: PCT/JP2022/041596
(87) International publication number: WO 2024/100776

(57) **Abstract**

A transformant obtained by introducing a chorismate-pyruvate lyase gene described in (a), (b), (c), (d), or (e) below into a *Hydrogenophilus* bacterium is capable of efficiently producing 4-hydroxybenzoic acid utilizing substantially only carbon dioxide as a carbon source.
(a) a DNA consisting of the nucleotide sequence of SEQ ID NO: 1 or 2
(b) a DNA consisting of a nucleotide sequence having 90% or more identity with SEQ ID NO: 1 or 2 and encoding a polypeptide having chorismate-pyruvate lyase activity
(c) a DNA encoding a polypeptide consisting of the amino acid sequence of SEQ ID NO: 3 or 4
(d) a DNA encoding a polypeptide consisting of an amino acid sequence having 90% or more identity with SEQ ID NO: 3 or 4 and having chorismate-pyruvate lyase activity
(e) a DNA encoding a polypeptide consisting of an amino acid sequence that has 1 to 5 amino acids deleted, substituted, inserted, or added in the amino acid sequence of SEQ ID NO: 3 or 4, and having chorismate-pyruvate lyase activity

## Description

### Technical Field

The present invention relates to a *Hydrogenophilus* bacterium transformant having ability to produce 4-hydroxybenzoic acid, and to a method for producing 4-hydroxybenzoic acid using the same.

### Background Art

The Paris Agreement, which was adopted in 2015, provides that global emissions of greenhouse gases should be promptly reduced. Under the Agreement, Japan set the goal of reducing her emission of greenhouse gases such as carbon dioxide and methane by 26% compared with year 2013 levels, by the year 2030.

Worldwide, the majority of the production of chemicals depends on petroleum resources, exacerbating the problem of increased greenhouse gas emissions. Accordingly, departure from petroleum dependency is a desirable strategy for the production of chemicals, and research and development of biorefineries that produce green chemicals from biomass is being earnestly carried out in various countries. However, the saccharification of biomass for use as raw materials of microbial fermentation necessitates complex processes, besides being costly. Using biomass that could serve as food or feed for chemical production disturbs the stable supply of food and feed. Furthermore, the large-scale consumption of biomass for chemical manufacturing raises concerns that it actually leads to environmental destruction.

As part of research geared towards departure from petroleum dependency, gases such as carbon dioxide, methane, and carbon monoxide have attracted attention as more sustainable carbon sources, and techniques for producing valuable chemicals and biofuels using microorganisms that utilize these gases are the subject of interest. In particular, carbon fixation and efficient utilization of carbon dioxide, a significant contributor to global warming, is highly anticipated.

Here, 4-hydroxybenzoic acid (hereinafter sometimes referred to as "4-HBA") is a raw material for liquid crystal polymers, dyes and pigments, paraben preservatives, plasticizers for nylon resins, biophenols, photographic chemicals, adhesives, fragrances, etc. and is a useful compound in great demand.

Currently, 4-HBA is chemically produced from crude oil. One method for chemically producing 4-HBA involves a reaction of potassium phenoxide, which is produced from phenol and potassium hydroxide, with carbon dioxide under pressurized and heated conditions. This method requires a complicated process and has high environmental load because it uses a large amount of phenol, which is synthesized from a petroleum-derived material, i.e., coal tar or benzene.

In this context, microbial fermentation-based production of 4-HBA using renewable biomass resources has been attracting attention as a way to help shift from a society dependent on petroleum resources, which are facing the risk of depletion in the future, to a sustainable society. However, as mentioned above, the use of biomass has various problems, such as requiring a complicated process, disturbing a stable supply of food and feed, and having high environmental load.

Therefore, there is a need for a practical method for producing 4-HBA using microorganisms in a simpler process without disturbing a stable supply of food and feed or causing environmental load. In particular, there is a need for a practical method that can produce 4-HBA through carbon dioxide fixation.

In microorganisms, 4-HBA is produced from chorismic acid on the shikimate pathway, which is involved in the synthesis of aromatic amino acids and other compounds, by the catalytic action of chorismate-pyruvate lyase. In addition, 4-HBA is produced from tyrosine by the catalytic actions of several enzymes.

Non Patent Literature 2 to 5 teach techniques for producing 4-HBA through fermentation with genetically modified microorganisms, and these techniques involve introducing a ubiC gene into *Escherichia coli* to induce high expression of chorismate-pyruvate lyase and producing 4-HBA from chorismic acid in the transformed *Escherichia coli.*

Patent Literature 1 discloses a method for producing 4-HBA, in which an *Escherichia coli* chorismate-pyruvate lyase gene is introduced into an *Escherichia coli* strain overexpressing chorismic acid.

Patent Literature 2 discloses a method for producing 4-HBA, in which an *Escherichia coli* chorismate-pyruvate lyase gene is introduced into *Escherichia coli* and the transformed *Escherichia coli* is cultured.

Patent Literature 3 discloses a method for producing 4-HBA, in which an *Escherichia coli* chorismate-pyruvate lyase gene is introduced into a phenylalanine high-producing *Escherichia coli* strain.

Non Patent Literature 6 to 9 disclose methods for producing 4-HBA, in which an *Escherichia coli* chorismate-pyruvate lyase gene is introduced into *Klebsiella pneumoniae, Pseudomonas putida, Corynebacterium glutamicum,* or *Nicotiana tabacum.*

Patent Literature 4 discloses a method for producing 4-HBA, in which a chorismate-pyruvate lyase gene of a *Rhodobacter* bacterium is introduced into *Escherichia coli* or a *Rhodobacter* bacterium. Patent Literature 4 is intended to obtain an industrially applicable 4-HBA high-producing strain by the introduction of a chorismate-pyruvate lyase gene compatible with a host.

Patent Literature 5 discloses successful production of 4-HBA in a culture supernatant of a *Corynebacterium glutamicum* transformant prepared by introducing a chorismate-pyruvate lyase gene of *Pantoea ananatis, Providencia rustigianii, Providencia stuartii, Escherichia coli,* or *Cronobacter sakazakii.*

However, these methods for producing 4-HBA use sugars, which are costly to produce, as a carbon source for growing microorganisms, and none of the methods use carbon dioxide as a carbon source.

Patent Literature 6 discloses a method for producing 4-HBA, in which an *Escherichia coli* chorismate-pyruvate lyase gene is introduced into an anaerobic *Clostridium* bacterium and the transformant is cultured. *Clostridium* bacteria use chlorine-containing gaseous substrates as a carbon source for their growth, but substantially require carbon monoxide as a carbon source to produce 4-HBA. Therefore, the method of Patent Literature 6 is not a method for producing 4-HBA using carbon dioxide as a carbon source, and contributes less to global warming countermeasures.

Among bacteria capable of using carbon dioxide as a carbon source for their growth, *Cupriavidus necator* H16 strain, *Hydrogenobacter thermophilus* TK-6 strain, and the like have been used for the production of chemical products, but there are no reports on using these strains as a host for 4-HBA production.

### Citation List

### Patent Literatures

[Patent Literature 1] US Patent 6,030,819
[Patent Literature 2] US Patent 6,114,157
[Patent Literature 3] WO2017/033965
[Patent Literature 4] JP2012-183048A
[Patent Literature 5] WO2015/156271
[Patent Literature 6] JP2018-522536A
[Non Patent Literature 1] Front Bioeng Biotechnol., 7, 130 (2019)
[Non Patent Literature 2] Biotechnol Bioeng., 76, 376-390(2001)
[Non Patent Literature 3] J Bacteriol., 174, 5309-5316(1992)
[Non Patent Literature 4] FEBS Lett., 307, 347-350(1992)
[Non Patent Literature 5] Microbiology (Reading)., 140m, 897-904 (1994)
[Non Patent Literature 6] Appl Microbiol Biotechnol., 43, 985-988 (1995)
[Non Patent Literature 7] Front Bioeng Biotechnol., 4, 90(2016)
[Non Patent Literature 8] Microb Cell Fact., 17, 70(2018)
[Non Patent Literature 9] Plant Physiol., 112, 811-819(1996)
[Non Patent Literature 10] Appl Environ Microbiol., 84(6): e02587-e02617 (2018)

### Summary of Invention

### Technical Problem

The objective of the present invention is to provide a transformant of a *Hydrogenophilus* bacterium that is capable of efficiently producing 4-HBA utilizing carbon dioxide as a sole carbon source, and a method for efficiently producing 4-HBA using this transformant.

### Solution to Problem

The inventor of the present invention has focused on *Hydrogenophilus* bacteria as bacteria capable of fixing carbon dioxide on an industrial scale for the purpose of avoiding the use of high cost raw materials such as sugars and contributing to global warming countermeasures. *Hydrogenophilus* bacteria are hydrogen-oxidizing bacteria which grow by producing organic substances from carbon dioxide by utilizing hydrogen energy. The growth rate of hydrogen-oxidizing bacteria is generally extremely slow, however, the growth rate of *Hydrogenophilus* bacteria is fast, and their ability of fixing carbon dioxide is remarkably higher than that of plants and photosynthetic bacteria.

*Hydrogenophilus* bacteria have a gene of chorismate-pyruvate lyase which produces 4-HBA from chorismic acid (ubiC gene). However, wild-type *Hydrogenophilus* bacteria are not able to produce 4-HBA on an industrial scale since they do not sufficiently produce chorismate-pyruvate lyase. Therefore, it is necessary to introduce the gene of an enzyme which catalyzes a reaction producing 4-HBA to *Hydrogenophilus* bacteria in order to provide capability of producing 4-HBA on an industrial scale to *Hydrogenophilus* bacteria.

The inventor of the present invention has found that when a heterologous gene is introduced into *Hydrogenophilus* bacteria using a vector that functions within the *Hydrogenophilus* bacteria, a functioning protein often is not produced or is insufficiently produced.

The inventor of the present invention also has found that when a heterologous gene which is expressed within bacteria other than *Hydrogenophilus* bacteria is introduced into *Hydrogenophilus* bacteria, a functioning protein often is not produced or is insufficiently produced. It is generally not practical to divert a gene which can produce substances within bacteria other than *Hydrogenophilus* bacteria, to *Hydrogenophilus* bacteria.

Under such circumstances, the present inventor aimed to obtain a chorismate-pyruvate lyase gene that is expressed in *Hydrogenophilus* bacteria and examined chorismate-pyruvate lyase genes of *Hydrogenophilus* bacteria and genes predicted to encode chorismate-pyruvate lyase in the genomes of various microorganisms. The present inventor has finally found that chorismate-pyruvate lyase genes of *Shewanella algae* and *Cronobacter sakazakii* allow the expression of functioning chorismate-pyruvate lyases in *Hydrogenophilus* bacteria. The present inventor has also found that the resulting transformants have the capability or potential to efficiently produce 4-HBA using carbon dioxide as a sole carbon source.

The present invention has been completed on the basis of the above findings and provides the following transformant and method for producing 4-HBA.
[1] A transformant obtained by introducing a chorismate-pyruvate lyase gene described in (a), (b), (c), (d), or (e) below into a *Hydrogenophilus* bacterium.
   (a) a DNA consisting of the nucleotide sequence of SEQ ID NO: 1 or 2
   (b) a DNA consisting of a nucleotide sequence having 90% or more identity with SEQ ID NO: 1 or 2 and encoding a polypeptide having chorismate-pyruvate lyase activity
   (c) a DNA encoding a polypeptide consisting of the amino acid sequence of SEQ ID NO: 3 or 4
   (d) a DNA encoding a polypeptide consisting of an amino acid sequence having 90% or more identity with SEQ ID NO: 3 or 4 and having chorismate-pyruvate lyase activity
   (e) a DNA encoding a polypeptide consisting of an amino acid sequence that has 1 to 5 amino acids deleted, substituted, inserted, or added in the amino acid sequence of SEQ ID NO: 3 or 4, and having chorismate-pyruvate lyase activity
[2] The transformant according to the above [1], wherein the *Hydrogenophilus* bacterium is *Hydrogenophilus thermoluteolus.*
[3] A method for producing 4-HBA, comprising a step of culturing the transformant according to the above [1] or [2] using substantially only carbon dioxide as a carbon source.

### Advantageous Effects of Invention

Measures to counter the increase in atmospheric carbon dioxide entail reduction of carbon dioxide emissions and fixation of emitted carbon dioxide. In order to reduce carbon dioxide emissions, solar, wind, geothermal, and similar energies are utilized in place of fossil energy. However, the utilization of such energies is not yet extensive enough to repress the buildup of atmospheric carbon dioxide. Consequently, there is need to enhance atmospheric carbon fixation or recycling of emitted carbon dioxide.

Carbon dioxide can be fixed through physical or chemical processes. However, fixation of carbon dioxide utilizing living cells allows the production of organic substances that can be used as food, feed, or fuel. In other words, carbon dioxide itself can be directly converted into valuable substances. Accordingly, the twin problems of global warming due to increased atmospheric carbon dioxide and scarcity of food, feed, and fuel can be solved. Further, in-demand chemical products can be produced while suppressing global warming attributed to increased carbon dioxide emissions.

4-HBA is an industrially important aromatic compound. However, the chemical synthesis of the aromatic compound 4-HBA, among other chemical products, often requires a large amount of energy and/or a large amount of solvent. For this reason, the chemical synthesis of 4-HBA is undesirable from the viewpoint of environmental protection. In contrast, the production of 4-HBA using carbon dioxide-fixing microorganisms can provide a solution to global warming caused by increased carbon dioxide emissions as well as to securing the supply of industrially necessary 4-HBA.

Bacteria that can grow by utilizing the chemical energy generated by the reaction of hydrogen with oxygen and by using carbon dioxide as a sole carbon source, can produce chemical products from a mixture of oxygen, hydrogen, and carbon dioxide gases as raw material. Therefore, the bacteria can achieve the efficient conversion of carbon dioxide into organic compounds and can be cultured in a simple culture medium. Typically, such bacteria grow slowly, but the growth rate of hydrogen-oxidizing bacteria, *Hydrogenophilus* bacteria is exceptionally high. The Journal of Mitsubishi Research Institute No. 34 1999 describes *Hydrogenophilus* bacteria as follows: "Their proliferative capacity is so high that their carbon dioxide fixation ability cannot be compared with that of plants, which truly indicates the high carbon dioxide fixation ability of microorganisms".

According to the present invention, introducing a specific chorismate-pyruvate lyase gene into *Hydrogenophilus* bacteria enables the expression of functional chorismate-pyruvate lyase within these bacteria to produce 4-HBA on an industrial scale.

As described above, *Hydrogenophilus* bacteria have an atypically remarkable carbon dioxide fixation ability among organisms having carbon dioxide fixation ability. Therefore, the present invention has established a pathway for the industrial-scale production of 4-HBA.

### Mode for Carrying Out the Invention

Hereinafter, the present invention will be described in detail.

### (1) Transformant capable of producing 4-HBA

The transformant according to the present invention is a transformant obtained by introducing a chorismate-pyruvate lyase (hereinafter sometimes abbreviated as "UbiC") gene of *Shewanella algae* or *Cronobacter sakazakii,* or a homologue thereof, into a host *Hydrogenophilus* bacterium. That is, the transformant according to the present invention is a *Hydrogenophilus* bacterium transformant having an exogenous UbiC gene, such as the UbiC gene of *Shewanella algae* or *Cronobacter sakazakii,* or a homologue thereof.

The UbiC gene used in the present invention does not have to be a known gene identified as a UbiC gene, and may be any DNA encoding a polypeptide having chorismate-pyruvate lyase activity. The chorismate-pyruvate lyase activity refers to an enzymatic activity to produce 4-HBA from chorismic acid.

In the present invention, the UbiC gene may be a DNA corresponding to a UbiC gene isolated from naturally occurring bacteria, or may be a DNA artificially synthesized using methods known to those skilled in the art.

### Chorismate-pyruvate lyase gene

The UbiC gene of *Shewanella algae* is a DNA consisting of the nucleotide sequence of SEQ ID NO: 1, and the UbiC gene of *Cronobacter sakazakii* is a DNA consisting of the nucleotide sequence of SEQ ID NO: 2.

In the present invention, a DNA consisting of a nucleotide sequence having 90% or more, even 95% or more, even 98% or more, even 99% or more identity with SEQ ID NO: 1 or 2 and encoding a polypeptide having chorismate-pyruvate lyase activity can also be used.

In the present invention, a DNA encoding a chorismate-pyruvate lyase of *Shewanella algae* or *Cronobacter sakazakii* can also be used. The chorismate-pyruvate lyase of *Shewanella algae* consists of the amino acid sequence of SEQ ID NO: 3, and the chorismate-pyruvate lyase of *Cronobacter sakazakii* consists of the amino acid sequence of SEQ ID NO: 4.

In addition, a DNA encoding a polypeptide consisting of an amino acid sequence having 90% or more, even 95% or more, even 98% or more, even 99% or more identity with SEQ ID NO: 3 or 4 and having chorismate-pyruvate lyase activity can also be used.

Furthermore, a DNA encoding a polypeptide consisting of an amino acid sequence that has 1 to 5, 1 to 4, 1 to 3, 1 to 2, or 1 amino acids deleted, substituted, inserted, or added in the amino acid sequence of SEQ ID NO: 3 or 4, and having chorismate-pyruvate lyase activity can also be used.

In the present invention, the identities of nucleotide sequences and amino acid sequences were calculated using GENETYX ver.17 (made by GENETYX Corporation).

In the present invention, the presence of chorismate-pyruvate lyase activity of a test polypeptide is determined by detecting an increase in absorbance at 246 nm, which is attributed to the production of 4-HBA by a reaction of the test polypeptide with 0.1 mM barium chorismate. Even a slight increase in absorbance at 246 nm confirms that the test polypeptide has chorismate-pyruvate lyase activity.

### Hydrogenophilus bacteria

Examples of *Hydrogenophilus* bacteria include *Hydrogenophilus thermoluteolus, Hydrogenophilus halorhabdus, Hydrogenophilus denitrificans, Hydrogenophilus hirschii, Hydrogenophilus islandicus, Hydrogenophilus* bacterium strain Mar3 (*Hydrogenophilus* sp. Mar3), and *Hydrogenophilus* bacterium strain Z1038 (*Hydrogenophilus* sp. Z1038). In particular, *Hydrogenophilus thermoluteolus* is preferable because its superior growth rate enables top-level cardon dioxide fixation among carbon dioxide fixing microorganisms.

*Hydrogenophilus* bacteria have been easily isolated from diverse regions everywhere on the earth. A preferable strain of *Hydrogenophilus thermoluteolus* is strain TH-1 (NBRC 14978). *Hydrogenophilus thermoluteolus* strain TH-1 (NBRC 14978) exhibits the highest rapid growth rate among carbon dioxide fixing microorganisms (Agricultural and Biological Chemistry, 41, 685-690 (1977)) (It proliferates double per hour.). *Hydrogenophilus thermoluteolus* strain NBRC 14978 is internationally deposited under the Budapest Treaty, and is thus publicly available.

The *Hydrogenophilus* bacteria into which the chorismate-pyruvate lyase gene is to be introduced in the present invention may be bacteria isolated from nature or genetically modified bacteria derived from bacteria isolated from nature. Genetic modification may be intended, for example, to allow high expression of an introduced chorismate-pyruvate lyase gene.

Such a genetic modification can be made, for example, by removing (curing) endogenous plasmids in *Hydrogenophilus* bacteria. Methods for removing endogenous plasmids are well known and include, for example, treating with chemicals such as novobiocin, SDS, acriflavine, and ethidium bromide; introducing a plasmid having the same origin of replication as those of endogenous plasmids to destabilize the endogenous plasmids; and utilizing the phenomenon of plasmid incompatibility, such as disrupting factors involved in the plasmid partition system to destabilize endogenous plasmids.

### Methods for producing transformants

Next, methods for obtaining transformants by introducing UbiC gene into *Hydrogenophilus* bacteria are described.

The introduction of UbiC gene into *Hydrogenophilus* bacteria can be carried out using common methods for introducing exogenous genes into bacteria. The UbiC gene may be directly introduced into *Hydrogenophilus* bacteria. Alternatively, it may be incorporated into a vector for transformation such as a plasmid vector, viral vector, cosmid, fosmid, BAC, or YAC, and then, an obtained vector may be introduced into *Hydrogenophilus* bacteria.

Vectors for transformation should contain a DNA which controls the autonomous replication function within *Hydrogenophilus* bacteria, and examples include broad-host-range vectors pRK415 (GenBank: EF437940.1), pBHR1 (GenBank: Y14439.1), pMMB67EH (ATCC 37622), pCAR1 (NCBI Reference Sequence: NC_004444.1), pC194 (NCBI Reference Sequence: NC_002013.1), pK18mobsacB (GenBank: FJ437239.1), pUB110 (NCBI Reference Sequence: NC_001384.1), vectors obtained by genetically modifying these vectors (For example, pCAMO-6), and the like.

Among them, pCAMO-6 is preferable. Those skilled in the art can prepare pCAMO-6 according to "Examples" of this specification.

Examples of promoters in vectors include tac promoter, lac promoter, trc promoter, or each of promoters OXB1 and OXB11 to OXB20 from Oxford Genetics Ltd. Examples of terminators in vectors include the T1T2 terminator of *Escherichia coli* rRNA operon rrnB, the t0 transcription terminator of bacteriophage λ, T7 terminator, and the like.

Introduction of chorismate-pyruvate lyase gene into a *Hydrogenophilus* bacterium (transformation) can be carried out by publicly known methods such as calcium chloride method, calcium phosphate method, DEAE-dextran transfection method, and electric pulse method (electroporation method).

### (2) Method for producing 4-HBA

The present invention provides a method for producing 4-HBA with use of the transformant of the present invention described above. The method includes a step of culturing the transformant of the present invention using an inorganic or organic culture medium while supplying a mixture of gases containing hydrogen, oxygen, and carbon dioxide. The supplied gas is preferably a mixture of gases consisting of hydrogen, oxygen, and carbon dioxide. However, different kinds of gases can be mixed within, to the extent that 4-HBA can be produced efficiently.

*Hydrogenophilus* bacteria can grow using hydrogen as a source of energy and using carbon dioxide as a sole carbon source, and thus, carbon dioxide can be fixed efficiently particularly by producing 4-HBA by using substantially only carbon dioxide (in particular, by using only carbon dioxide) as a carbon source. Therefore, using an inorganic culture medium that does not contain carbon sources such as organic substances and carbonates, namely, carrying out culture using substantially only carbon dioxide (in particular, using only carbon dioxide) as a carbon source is preferable. "Using substantially only carbon dioxide as a carbon source" encompasses cases in which an unavoidable amount of other carbon sources is mixed within.

The pH of the culture medium is preferably 6.2 to 8, more preferably 6.4 to 7.4, and furthermore preferably 6.6 to 7. When the pH is within this range, bacteria grow well and mixed gases dissolves well into the culture medium, and 4-HBA can be produced efficiently.

When batch culture is utilized, mixed gases can be entrapped within an airtight culture container and static culture or shaking culture can be carried out. When continuous culture is utilized, mixed gases can be continuously supplied into an airtight culture container and shaking culture can be carried out, or the transformant can be cultured using an airtight culture container while introducing mixed gases into the culture medium by bubbling. Shaking culture is preferable in that better dissolution of mixed gases into the culture medium can be achieved.

The volume ratio of hydrogen, oxygen, and carbon dioxide (hydrogen: oxygen: carbon dioxide) in the supplied gas mixture is preferably 1.75 to 7.5:1:0.25 to 3, more preferably 5 to 7.5:1:1 to 2, and even more preferably 6.25 to 7.5:1:1.5. When the volume ratio is within this range, bacteria grow well, and 4-HBA can be produced efficiently.

The supply rate of mixed gases or raw material gases can be 10 to 60 L/hour, in particular 10 to 40 L/hour, in particular 10 to 20 L/hour, per 1 L of culture medium. When the supply rate is within this range, transformants grow well and 4-HBA can be produced efficiently, and the amount of wasted mixed gases can be reduced.

The culture temperature is preferably 35 to 55°C, more preferably 37 to 52°C, and even more preferably 50 to 52°C. When the temperature is within this range, transformants grow well, and 4-HBA can be produced efficiently.

4-HBA is produced in the medium solution by culturing in the above-described manner. Collecting the medium solution will enable the recovery of 4-HBA, however, 4-HBA can furthermore be separated from the medium solution by publicly known methods. Such publicly known methods include ionexchange resin method, concentration method, crystallization method, membrane separation method, organic solvent extraction method, various adsorption methods, and the like.

### Examples

The present invention will now be described with reference to examples. The technical scope of the present invention is not limited by the following examples.

### (1) Construction of plasmid vector (pCAMO-6)

The method for constructing a pCAMO-6 plasmid vector used for the introduction of a chorismate-pyruvate lyase (UbiC) gene is described below.

### (1-1) Preparation of tac promoter-containing DNA fragment

A pMAL-c5X plasmid manufactured by New England Biolabs was used as a template to amplify a DNA fragment containing a tac promoter by PCR using a pair of primers shown below. PCR was performed in the usual manner using a 2720 Thermal Cycler manufactured by Thermo Fisher Scientific Inc. and using a KOD FX Neo (manufactured by Toyobo Co., Ltd.) as a reaction reagent.
Primers for amplification of tac promoter
(a-1) 5'-TTTTATAACCCGGGCCATCGACTGCACGGTGCACC-3' (SEQ ID NO: 5)
(b-1) 5'-TGCTAGCACTGTTTCCTGTGTGAAATTGTTATCCG-3' (SEQ ID NO: 6)

The primer (a-1) contains a SmaI restriction enzyme site as indicated by underlining.

The resulting reaction mixture was subjected to electrophoresis on a 1% agarose gel. The results showed that an about 0.3-kbp DNA fragment corresponding to the tac promoter was detected.

### (1-2) Preparation of multicloning site-containing DNA fragment

For the preparation of a DNA fragment containing a multicloning site, the former and latter halves of the multicloning site were separately produced by PCR using pairs of primers shown below. PCR was performed in the usual manner using a DNA Thermal Cycler manufactured by Life Technologies Inc. and using a KOD FX Neo (manufactured by Toyobo Co., Ltd.) as a reaction reagent. This method does not use any template DNA and allows each pair of primers to anneal together in their 3' terminal regions and elongate to form a double-stranded DNA.
Primers for preparation of former half of multicloning site
(a-2) 5'-GGAAACAGTGCTAGCAGATCTGGAGGAGAAAGGCATAT-3' (SEQ ID NO: 7)
(b-2) 5'-CAGTGCGGCCGCAAGCTTGTCGACGGAGCTCGAATTCGGATCCGATATCAGCATATGCGTTTC TCCTCCAGA-3' (SEQ ID NO: 8)

The 3' terminal nucleotide sequences of the primers (a-2) and (b-2) are complementary to each other.
Primers for preparation of latter half of multicloning site
(a-3) 5'-ACAAGCTTGCGGCCGCACTGCAGCACCATCACCACCATCATTGATAAGATCCGGCTGCTAACA AAGCCCGAAAGGAAGCT-3' (SEQ ID NO: 9)
(b-3) 5'-TATTTGAATCGAGTTATTGCTCAGCGGTGGCAGCAGCCAACTCAGCTTCCTTTCGGGCTTTGT -3' (SEQ ID NO: 10)

The 3' terminal nucleotide sequences of the primers (a-3) and (b-3) are complementary to each other.

The resulting reaction mixture was subjected to electrophoresis on a 1% agarose gel. The results showed that about 0.1-kbp DNA fragments corresponding to the former and latter halves of the multicloning site were detected. Each gel portion containing the DNA fragment was cut out of the agarose gel, and the gel portion was freeze-thawed to recover the DNA fragment.

Overlap extension PCR was performed through use of the recovered DNA fragments corresponding to the former and latter halves of the multicloning site as a template. The 5' terminal nucleotide sequences of the primers (b-2) and (a-3) used to amplify these template DNA fragments are complementary to each other. For the overlap extension PCR, a combination of the primers (a-2) and (b-3) shown above was used to construct a DNA containing the multicloning site. PCR was performed in the usual manner using a DNA Thermal Cycler manufactured by Life Technologies Inc. and using a KOD FX Neo (manufactured by Toyobo Co., Ltd.) as a reaction reagent.

The resulting reaction mixture was subjected to electrophoresis on a 1% agarose gel. The results showed that an about 0.2-kbp DNA fragment corresponding to the multicloning site was detected.

### (1-3) Preparation of DNA fragment containing rrnB terminator joined to origin of replication of pUC19

A pMAL-c5X plasmid manufactured by New England Biolabs was used as a template to amplify a DNA fragment containing an rrnB terminator by PCR using a pair of primers shown below.
Primers for amplification of rrnB terminator
(a-4) 5'-TAACTCGATTCAAATAAAACGAAAGGCTCAGTCGA-3' (SEQ ID NO: 11)
(b-4) 5'-CCTAGATCCGCGGAGTTTGTAGAAACGCAAAAAGG-3' (SEQ ID NO: 12)

In addition, a pUC19 plasmid was used as a template to amplify a DNA fragment containing an origin of DNA replication by PCR using a pair of primers shown below.
Primers for amplification of origin of DNA replication of pUC19
(a-5) 5'-ACAAACTCCGCGGATCTAGGTGAAGATCCTTTTTG-3' (SEQ ID NO: 13)
(b-5) 5'-CGTCCGCGGCCAGCAAAAGGCCAGGAACCGTAAAA-3' (SEQ ID NO: 14)

Each PCR was performed in the usual manner using a DNA Thermal Cycler manufactured by Life Technologies Inc. and using a KOD FX Neo (manufactured by Toyobo Co., Ltd.) as a reaction reagent.

Each resulting reaction mixture was subjected to electrophoresis on a 1% agarose gel. The results showed that an about 0.3-kbp DNA fragment corresponding to the rrnB terminator and an about 0.8-kbp DNA fragment corresponding to the origin of DNA replication of pUC19 were detected. Each gel portion containing the DNA fragment was cut out of the agarose gel, and the gel portion was freeze-thawed to recover the DNA fragment.

Overlap extension PCR was performed through use of the recovered DNA fragments corresponding to the rrnB terminator and the origin of DNA replication of pUC19 were used as templates. The 5' terminal nucleotide sequences of the primers (b-4) and (a-5) used to amplify these template DNA fragments are complementary to each other. For the overlap extension PCR, a combination of the primers (a-4) and (b-5) was used to construct a DNA fragment containing the rrnB terminator joined to the origin of replication of pUC19. PCR was performed in the usual manner using a DNA Thermal Cycler manufactured by Life Technologies Inc. and using a KOD FX Neo (manufactured by Toyobo Co., Ltd.) as a reaction reagent.

The resulting reaction mixture was subjected to electrophoresis on a 1% agarose gel. The results showed that an about 1.0-kbp DNA fragment corresponding to a DNA containing the rrnB terminator joined to the origin of replication of pUC19 was detected.

### (1-4) Preparation of DNA fragment containing neomycin/kanamycin resistance gene

A pK18mobsacB plasmid (GenBank: FJ437239.1) (Gene, 145, 69-73 (1994)), which contains a neomycin/kanamycin resistance gene (hereinafter sometimes referred to as "nptII") sequence, was used as a template for conventional PCR. For the amplification of a DNA fragment containing the nptII gene sequence, a pair of primers shown below was used for PCR. PCR was performed in the usual manner using a DNA Thermal Cycler manufactured by Life Technologies Inc. and using a KOD FX Neo (manufactured by Toyobo Co., Ltd.) as a reaction reagent.
Primers for amplification of nptII gene
(a-6) 5'-TTGCTGGCCGCGGACGTAGAAAGCCTGTCCGCAGA-3' (SEQ ID NO: 15)
(b-6) 5'-GGCCCGGGTTATAAAAGCCAGTCATTAGGCCTATC-3' (SEQ ID NO: 16)

The primer (b-6) contains a SmaI restriction enzyme site as indicated by underlining.

The resulting reaction mixture was subjected to electrophoresis on a 1% agarose gel. The results showed that an about 1.0-kbp DNA fragment corresponding to the nptII gene was detected.

### (1-5) Construction of circular plasmid

For the DNA fragments prepared in the above (1-1) to (1-4), the terminal region of the DNA fragment in the above (1-1) has a 16-bp sequence homologous to the terminal regions of the DNA fragments in the above (1-4) and (1-2); the terminal region of the DNA fragment in the above (1-2) has a 16-bp sequence homologous to the terminal regions of the DNA fragments in the above (1-1) and (1-3); the terminal region of the DNA fragment in the above (1-3) has a 16-bp sequence homologous to the terminal regions of the DNA fragments in the above (1-2) and (1-4); and the terminal region of the DNA fragment in the above (1-4) has a 16-bp sequence homologous to the terminal regions of the DNA fragments in the above (1-3) and (1-1).

Thus, the DNA fragments in the above (1-1), (1-2), (1-3), and (1-4) can be joined together into the form of a circular DNA by Gibson Assembly. Gibson Assembly was performed using a Gibson Assembly Master Mix manufactured by New England Biolabs to join the DNA fragments prepared in the above (1-1) to (1-4) into a circular DNA. With the resulting reaction mixture, *Escherichia coli* JM109 was transformed according to the calcium chloride method, and applied to a solid LB medium containing 50 µg/mL kanamycin. The grown strain on the medium was cultured in liquid medium in the usual manner. Plasmid DNA was extracted from the culture liquid and reacted with a restriction enzyme, SmaI. This reaction mixture was subjected to electrophoresis on a 1% agarose gel. The results showed that an about 2.3-kbp DNA fragment was observed, which corresponds in size to a single piece of DNA formed by joining the DNA fragments prepared in the above (1-1) to (1-4), demonstrating successful assembly of the DNA fragments.

This circular plasmid DNA, which is replicable in *Escherichia coli,* was named pCAMO-1.

### (1-6) Construction of plasmid vector (pCAMO-4)

*Hydrogenophilus thermoluteolus* TH-1 strain was inoculated with a platinum loop into 5 mL of liquid A medium [3.0 g of (NH₄)₂SO₄, 1.0 g of KH₂PO₄, 2.0 g of K₂HPO₄, 0.25 g of NaCl, 0.014 g of FeSO₄·7H₂O, 0.5 g of MgSO₄·7H₂O, 0.03 g of CaCl₂, 4.0 mg of MoO₃, 28 mg of ZnSO₄·7H₂O, 2.0 mg of CuSO₄·5H₂O, 4.0 mg of H₃BO₃, 4.0 mg of MnSO₄·5H₂O, and 4.0 mg of CoCl₂·6H₂O were dissolved in 1 L of distilled water (pH 7.0)]in a test tube. The test tube was filled with a gas mixture of H₂:O₂:CO₂ = 7.5:1:1.5, and the strain was cultured at 50°C.

An endogenous plasmid, pTH1 (Microbiol Resour Announc. 2018 Aug 16; 7(6)), was prepared from the culture liquid according to the conventional alkaline-SDS method.

The prepared pTH-1 of about 66 kbp in size was cut with restriction enzymes ScaI and PvuII, and the pCAMO-1 plasmid was cut with the restriction enzyme SmaI. Both of them were ligated together by T4 DNA (manufactured by Takara Bio Inc.).

With the resulting ligation mixture, *Hydrogenophilus thermoluteolus* TH-1 strain (NBRC 14978) was transformed according to the electric pulse method (electroporation), applied to solid A medium [3.0 g of (NH₄)₂SO₄, 1.0 g of KH₂PO₄, 2.0 g of K₂HPO₄, 0.25 g of NaCl, 0.014 g of FeSO₄·7H₂O, 0.5 g of MgSO₄·7H₂O, 0.03 g of CaCl₂, 4.0 mg of MoO₃, 28 mg of ZnSO₄·7H₂O, 2.0 mg of CuSO₄·5H₂O, 4.0 mg of H₃BO₃, 4.0 mg of MnSO₄·5H₂O, 4.0 mg of CoCl₂·6H₂O, and 15 g of agar were dissolved in 1 L of distilled water (pH 7.0)] containing 50 µg/mL kanamycin, and cultured in a chamber filled with a gas mixture of H₂:O₂:CO₂ = 7.5:1:1.5 at 50°C for 60 hours.

Each of seven strains grown on the solid A medium was inoculated with a platinum loop into 5 mL of liquid A medium containing 50 µg/mL kanamycin in a test tube. The test tubes were filled with a gas mixture of H₂:O₂:CO₂ = 7.5:1:1.5, and the strains were cultured with shaking at 50°C. Plasmid DNA was extracted from the culture and subjected to electrophoresis on a 1% agarose gel. The results showed that all of the 7 strains had the same plasmid DNA of about 5.5 kbp in size.

The extracted plasmid was used as a template to amplify a DNA fragment containing the endogenous plasmid pTH1 inserted in the SmaI restriction enzyme site of pCAMO-1 by PCR using a pair of primers shown below. The pair of primers correspond to the regions at both sides of the SmaI restriction enzyme site of pCAMO-1. PCR was performed in the usual manner using a DNA Thermal Cycler manufactured by Life Technologies Inc. and using a KOD FX Neo (manufactured by Toyobo Co., Ltd.) as a reaction reagent.
Primers for amplification of inserted pTH1 DNA fragment
(a-7) 5'-AATTGTCAGATAGGCCTAATGACTGGCTTTTATAA-3' (SEQ ID NO: 17)
(b-7) 5'-TGACGCCAGAAGCATTGGTGCACCGTGCAGTCGAT-3' (SEQ ID NO: 18)

The resulting reaction mixture was subjected to electrophoresis on a 1% agarose gel. The results showed that an about 3.2-kbp DNA fragment was detected. That is, the obtained plasmid contains an about 3.2-kbp DNA fragment corresponding to a part of pTH1, which has been inserted into the SmaI restriction enzyme site of the pCAMO-1 plasmid. This about 3.2-kbp DNA fragment contains a origin of replication that functions in *Hydrogenophilus thermoluteolus* cells, so that the obtained plasmid replicates in *Hydrogenophilus thermoluteolus* cells.

The constructed plasmid was named pCAMO-4.

### (1-7) Construction of plasmid vector (pCAMO-5)

Equal moles of a pair of oligonucleotides shown below were mixed, and the mixture was gently cooled from 98°C to 20°C, thus yielding a double-stranded DNA in which the oligonucleotides were annealed. Both ends of this DNA fragment are equivalent to the protruding ends generated by cleavage with restriction enzymes BglII and NdeI. This DNA fragment and a digested DNA fragment prepared by cutting pCAMO-4 with restriction enzymes BglII and NdeI were ligated together by T4 DNA ligase (manufactured by Takara Bio Inc.).
(a-8) 5'-GATCTGGAGGAGAAACGCA-3' (SEQ ID NO: 19)
(b-8) 5'-TATGCGTTTCTCCTCCA-3' (SEQ ID NO: 20)

The constructed plasmid was named pCAMO-5.

### (1-8) Construction of plasmid vector (pCAMO-6)

The endogenous plasmid pTH-1 was used as a template to amplify a DNA fragment containing a mazF gene encoding a plasmid stabilization factor by PCR using a pair of primers shown below. PCR was performed in the usual manner using a DNA Thermal Cycler manufactured by Life Technologies Inc. and using a KOD FX Neo (manufactured by Toyobo Co., Ltd.) as a reaction reagent.
Primers for amplification of mazF gene
(a-9) 5'-GAGGCCATCTAGGCCATGAGTAAGTCTGACGGAAC-3' (SEQ ID NO: 21)
(b-9) 5'-ATCCGGCACCCATATCTGAACCGGACGCAAACCCG-3' (SEQ ID NO: 22)

The endogenous plasmid pTH-1 was used as a template to amplify a DNA fragment containing a mazE gene encoding a plasmid stabilization factor by PCR using a pair of primers shown below. PCR was performed in the usual manner using a DNA Thermal Cycler manufactured by Life Technologies Inc. and using a KOD FX Neo (manufactured by Toyobo Co., Ltd.) as a reaction reagent.
Primers for amplification of mazE gene
(a-10) 5'-TTCAGATATGGGTGCCGGATACCCGCCGCCCGGGC-3' (SEQ ID NO: 23)
(b-10) 5'-AAGGCCTTCATGGCCTTATTTCGCGATTCCCAAGA-3' (SEQ ID NO: 24)

The 3' terminal region of the mazF-containing DNA fragment has a 20-bp sequence homologous to the 5' terminal region of the mazE-containing DNA fragment. Thus, the mazF-containing DNA fragment and the mazE-containing DNA fragment can be joined by PCR. The DNA fragments prepared above were mixed, and for the amplification of the joined DNA fragments, the primers (a-9) and (b-10) were used. PCR was performed in the usual manner using a DNA Thermal Cycler manufactured by Life Technologies Inc. and using a KOD FX Neo (manufactured by Toyobo Co., Ltd.) as a reaction reagent.

The resulting reaction mixture was subjected to electrophoresis on a 1% agarose gel. The results showed that an about 0.7-kbp DNA fragment corresponding to a DNA formed by joining the mazF-containing DNA fragment and the mazE-containing DNA fragment was detected.

The prepared DNA fragment of about 0.7 kbp in size was cut with a restriction enzyme, SfiI, and the pCAMO-5 plasmid was cut with the restriction enzyme SfiI. Both fragments were ligated together by T4 DNA ligase (manufactured by Takara Bio Inc.).

The constructed plasmid was named pCAMO-6.

### (2) Construction of expression plasmids for UbiC genes

Expression plasmids for the UbiC genes were constructed by, what is called, seamless cloning, a method for cloning a target DNA into a vector through recombination between homologous sequences.

DNA fragments containing UbiC genes from different bacteria shown below were amplified by PCR.
*Shewanella algae* (SEQ ID NO: 1)
*Cronobacter sakazakii* SEQ ID NO: 2)
*Aquitalea magnusonii* (SEQ ID NO: 25)
*Cupriavidus necator* H16 (SEQ ID NO: 26)
*Mixta theicola* (SEQ ID NO: 27)
*Proteus mirabilis* (SEQ ID NO: 28)
*Pseudomonas fluorescens* (SEQ ID NO: 29)
*Azotobacter vinelandii* (SEQ ID NO: 30)
*Hydrogenophilus thermoluteolus* (SEQ ID NO: 31)

The primers shown below were used for PCR. PCR was performed in the usual manner using a DNA Thermal Cycler manufactured by Life Technologies Inc. and using a KOD One PCR Master Mix (manufactured by Toyobo Co., Ltd.) as a reaction reagent.
Primers for amplification of *Shewanella algae* UbiC gene
(a-11) 5'-GCAGATCTGGAGGAGAAACGCATATGAGTGTGACCAGTTTAAGC-3' (SEQ ID NO: 32)
(b-11) 5'-CTTGTCGACGGAGCTCGAATTCTCAAGGCGTAAGTTGGCCTTG-3' (SEQ ID NO: 33)

The primers (a-11) and (b-11) contain their respective sequences homologous to sequences in the pCAMO-6 vector.
Primers for amplification of *Cronobacter sakazakii* UbiC gene
(a-12) 5'-CAGATCTGGAGGAGAAACGCATATGTCCCATCCCGCGCTGAGAC-3' (SEQ ID NO: 34)
(b-12) 5'-GCTTGTCGACGGAGCTCGAATTCTTATTTTTCCTCTCCGTAC-3' (SEQ ID NO: 35)

The primers (a-12) and (b-12) contain their respective sequences homologous to sequences in the pCAMO-6 vector.
Primers for amplification of *Aquitalea magnusonii* UbiC gene
(a-13) 5'-CAGATCTGGAGGAGAAACGCATATGTCCGGCTTTGGCATAATGC-3' (SEQ ID NO: 36)
(b-13) 5'-CTTGTCGACGGAGCTCGAATTCTCATCGGGCAAAATCTTTCAG-3' (SEQ ID NO: 37)

The primers (a-13) and (b-13) contain their respective sequences homologous to sequences in the pCAMO-6 vector.
Primers for amplification of *Cupriavidus necator* H16 UbiC gene
(a-14) 5'-GATCTGGAGGAGAAACGCATATGAGCGCGCAGTCCGCGCGC-3' (SEQ ID NO: 38)
(b-14) 5'-GTCGACGGAGCTCGAATTCTCATCTCGTGGTCTCTTTCTTG-3' (SEQ ID NO: 39)

The primers (a-14) and (b-14) contain their respective sequences homologous to sequences in the pCAMO-6 vector.
Primers for amplification of *Mixta theicola* UbiC gene
(a-15) 5'-GATCTGGAGGAGAAACGCATATGTCCGATAACGCGCTTTCCCTG-3' (SEQ ID NO: 40)
(b-15) 5'-GTCGACGGAGCTCGAATTCTTACTCACTTGGCTCTCCAGGCAG-3' (SEQ ID NO: 41)

The primers (a-15) and (b-15) contain their respective sequences homologous to sequences in the pCAMO-6 vector.
Primers for amplification of *Proteus mirabilis* UbiC gene
(a-16) 5'-GATCTGGAGGAGAAACGCATATGTTTAAAAAATCCATAATTAC-3' (SEQ ID NO: 42)
(b-16) 5'-GCTTGTCGACGGAGCTCGAATTCCTACTGCTTATATACAGGTG-3' (SEQ ID NO: 43)

The primers (a-16) and (b-16) contain their respective sequences homologous to sequences in the pCAMO-6 vector.
Primers for amplification of *Pseudomonas fluorescens* UbiC gene
(a-17) 5'-CAGATCTGGAGGAGAAACGCATATGCCGCACTCAATCGCCCTTC-3' (SEQ ID NO: 44)
(b-17) 5'-GCTTGTCGACGGAGCTCGAATTCTCAGCAGTTCTCCGGATGGTC-3' (SEQ ID NO: 45)

The primers (a-17) and (b-17) contain their respective sequences homologous to sequences in the pCAMO-6 vector.
Primers for amplification of *Azotobacter vinelandii* UbiC gene
(a-18) 5'-CAGATCTGGAGGAGAAACGCATATGACCGCTGCTCCCGCTTTC-3' (SEQ ID NO: 46)
(b-18) 5'-CTTGTCGACGGAGCTCGAATTCTTATAGGGTGTCCGGGTCG-3' (SEQ ID NO: 47)

The primers (a-18) and (b-18) contain their respective sequences homologous to sequences in the pCAMO-6 vector.
Primers for amplification of *Hydrogenophilus thermoluteolus* TH-1 UbiC gene
(a-19) 5'-CAGATCTGGAGGAGAAACGCATATGCGTGAGGCATCCGCTGCTG-3' (SEQ ID NO: 48)
(b-19) 5'-CTTGTCGACGGAGCTCGAATTCTCATTCTACCGGGTTCACAGCG-3' (SEQ ID NO: 49)

The primers (a-19) and (b-19) contain their respective sequences homologous to sequences in the pCAMO-6 vector.

The resulting reaction mixtures were subjected to electrophoresis on a 1% agarose gel. The results showed that about 0.7-kbp fragments corresponding to the UbiC genes of the respective bacterial strains were detected.

Each of gel portions containing the DNA fragment corresponding to the UbiC gene was cut out of the agarose gel, and the DNA fragments containing the UbiC genes were recovered from the gel portions with a GEL/PCR Purification Mini Kit (FAVORGEN).

The pCAMO-6 plasmid vector was amplified by PCR for subsequent seamless cloning. The primers shown below were used for PCR. PCR was performed in the usual manner using a DNA Thermal Cycler manufactured by Life Technologies Inc. and using a KOD One PCR Master Mix (manufactured by Toyobo Co., Ltd.) as a reaction reagent.
Primers for amplification of pCAMO-6 plasmid vector
(a-20) 5'-GAATTCGAGCTCCGTCGACA-3' (SEQ ID NO: 50)
(b-20) 5'-ATGCGTTTCTCCTCCAGATC-3' (SEQ ID NO: 51)

The gel portion containing a DNA fragment corresponding to the vector gene was cut out of the agarose gel, and the DNA fragment containing the vector gene was recovered from the gel portion with a GEL/PCR Purification Mini Kit (FAVORGEN).

The DNA fragment containing the pCAMO-6 vector and the DNA fragments containing the UbiC genes as synthesized above were joined together by a recombinase extracted from *Escherichia coli* JM109 strain.

With the resulting reaction mixtures, *Escherichia coli* JM109 strain was transformed by heat shock, applied to LB media with 50 µg/mL kanamycin, and cultured at 37°C for 24 hours.

Each of the strains grown on the LB media was inoculated with a platinum loop into 5 mL of liquid LB medium containing 50 µg/mL kanamycin in a test tube, and cultured with shaking at 37°C. Plasmid DNA was extracted from the culture. The sequence of the UbiC gene inserted in each plasmid was analyzed according to the Sanger method by Eurofins Genomics K.K. under contract, and confirmed to be identical to the corresponding sequence in the database.

### (3) Transformants of Hydrogenophilus thermoluteolus

### (3-1) Gene transfer

*Hydrogenophilus thermoluteolus* TH-1 strain was transformed with the obtained plasmids according to the electric pulse method (electroporation), applied to solid LB media containing 50 µg/mL kanamycin, and cultured at 52°C for 24 hours. The strains grown on the solid LB media were inoculated with a platinum loop on separate solid LB media containing 50 µg/mL kanamycin, and cultured at 52°C for 24 hours. The grown strains on the solid LB media were examined to confirm whether the inserted fragments in the respective plasmids would be amplified by PCR. Each PCR was performed in the usual manner using a DNA Thermal Cycler manufactured by Life Technologies Inc. and using a KOD One PCR Master Mix (manufactured by Toyobo Co., Ltd.) as a reaction reagent.

The results showed that DNA fragments of about 0.7-kbp in size corresponding to the respective UbiC genes were amplified. The plasmids containing the UbiC genes of the respective bacteria and the *Hydrogenophilus thermoluteolus* TH-1 transformants with the respective plasmids were named as shown in Table 1.

### (3-2) Measurement of chorismate-pyruvate lyase activity

The transformed strains of *Hydrogenophilus thermoluteolus* with the respective UbiC genes as prepared above were inoculated with a platinum loop into separate liquid A media containing 50 µg/mL kanamycin, and cultured with shaking at 52°C for 24 hours while a gas mixture of H₂:O₂:CO₂ = 7.5:1:1.5 was supplied. The cultured bacterial cells were collected by centrifugation (4°C, 5,000 g, 10 minutes). The bacterial cells were disrupted by sonication and then centrifuged (4°C, 20,000 g, 3 minutes) to obtain the supernatants of the disrupted cells.

Chorismate-pyruvate lyase activity was measured using the disrupted cell supernatants as crude enzyme solutions. That is, these crude enzyme solutions were separately mixed with barium chorismate for a reaction to proceed at 52°C. The increase in absorbance at 246 nm, which is attributed to the production of 4-HBA, was monitored, and the initial rate of the reaction was analyzed. Chorismate-pyruvate lyase activity was calculated from the initial rate of the reaction.

As shown in Table 1, chorismate-pyruvate lyase activity was observed in the culture supernatants of the UbiC01 transformed strain with the *Shewanella algae* UbiC gene and the UbiC02 transformed strain with the *Cronobacter sakazakii* UbiC gene.

### (3-3) Production of 4-HBA

The transformed strains of *Hydrogenophilus thermoluteolus* with the respective UbiC genes as prepared above were inoculated with a platinum loop into separate liquid A media containing 50 µg/mL kanamycin, and cultured with shaking at 52°C for 24 to 72 hours while a gas mixture of H₂:O₂:CO₂ = 7.5:1:1.5 was supplied. After that, culture supernatants were collected by centrifugation (4°C, 5,000 g, 10 minutes). The concentration of 4-HBA in the culture supernatants was determined using a high-performance liquid chromatography system (Shimadzu Corporation) under the following conditions.
Mobile phase A: 5 mmol/L p-toluenesulfonic acid:ethanol 90:10 (v/v)
Mobile phase B: 5 mmol/L p-toluenesulfonic acid, 20 mmol/L Bis-Tris, 0.1 mmol/L EDTA:ethanol 90:10 (v/v)
Flow rate: 0.7 mL/min for both mobile phases A and B
Column: Shim-pack SCR-102H
Column temperature: 45°C
Detector: CDD-10A VP
Injection sample volume: 20 µL

As shown in Table 1, 0.5 mM 4-HBA was detected in the culture supernatant of the UbiC01 transformed strain with the *Shewanella algae* UbiC gene. On the other hand, 4-HBA was not detected in the UbiC02 transformed strain with the *Cronobacter sakazakii* UbiC gene, but this strain would produce a detectable level of 4-HBA under different culture conditions.

The transformed strains with the UbiC genes of *Shewanella algae* and *Cronobacter sakazakii* were shown to be capable of producing 4-HBA, unlike other transformed strains with the UbiC genes of other bacteria and strains not transformed with any UbiC gene.

**[Table 1]**

| Strain | Origin of UbiC gene | Chorismate-pyruvate lyase activity (U/mg protein) | Concentration of 4-HBA in medium supernatant (mM) |
|---|---|---|---|
| UbiC01 | Shewanella algae | 0.025 | 0.5 |
| UbiC02 | Cronobacter sakazakii | 0.012 | ND |
| UbiC03 | *Aquitalea magnusonii* | ND | ND |
| UbiC04 | *Cupriavidus necator* H16 | ND | ND |
| UbiC05 | *Mixta theicola* | ND | ND |
| UbiC06 | *Proteus mirabilis* | ND | ND |
| UbiC07 | *Pseudomonas fluorescens* | ND | ND |
| UbiC08 | *Azotobacter vinelandii* | ND | ND |
| UbiC09 | *Hydrogenophilus thermoluteolus* | ND | ND |
| p-CAMO-6/TH-1 | - | ND | ND |

| | | | |
|---|---|---|---|
| 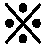 ND: Not Detected | | | |

The UbiC genes in the transformed strains shown not to have chorismate-pyruvate lyase activity as detectable in the culture supernatant were highly (i.e., 50 to 100%) identical to the UbiC genes reportedly effective for enhancing the production of chorismate-pyruvate lyase when introduced into microorganisms other than *Hydrogenophilus* bacteria (except for the UbiC genes of *Aquitalea magnusonii* and *Hydrogenophilus thermoluteolus*). However, when these genes were introduced into *Hydrogenophilus* bacteria, no functioning chorismate-pyruvate lyase was expressed in the bacteria. This demonstrates that it is not easy to search for UbiC genes expressible in *Hydrogenophilus* bacteria among UbiC genes expressible in other microorganisms.

The *Hydrogenophilus* bacterium transformant of the present invention can be prepared by referring to the description of "Examples" of this specification. Other strains mentioned in this specification are publicly available, as they are internationally deposited under the Budapest Treaty, or are possessed by organizations that furnish the strains without any restriction, or are marketed, or can be prepared by those skilled in the art based on this specification.

### Industrial Applicability

The transformant according to the present invention can highly efficiently produce 4-HBA using carbon dioxide as a sole carbon source, thus providing a solution to global warming caused by increased carbon dioxide emissions and contributing to high-efficient industrial production of chemical products.

That is, due to the atypically remarkable carbon dioxide fixation ability of *Hydrogenophilus* bacteria among organisms having carbon dioxide fixation ability, using the transformant according to the present invention enables industrial-scale 4-HBA production through carbon dioxide fixation. In addition, since 4-HBA is a raw material for liquid crystal polymers, dyes and pigments, paraben preservatives, plasticizers for nylon resins, biophenols, photographic chemicals, adhesives, fragrances, etc., the present invention also enables industrial production of products made from 4-HBA as a raw material using only carbon dioxide, without the use of sugars, which are costly to produce, or petroleum-derived raw materials such as phenol, which have high environmental load.

## Claims

1. A transformant obtained by introducing a chorismate-pyruvate lyase gene described in (a), (b), (c), (d), or (e) below into a *Hydrogenophilus* bacterium.
(a) a DNA consisting of the nucleotide sequence of SEQ ID NO: 1 or 2
(b) a DNA consisting of a nucleotide sequence having 90% or more identity with SEQ ID NO: 1 or 2 and encoding a polypeptide having chorismate-pyruvate lyase activity
(c) a DNA encoding a polypeptide consisting of the amino acid sequence of SEQ ID NO: 3 or 4
(d) a DNA encoding a polypeptide consisting of an amino acid sequence having 90% or more identity with SEQ ID NO: 3 or 4 and having chorismate-pyruvate lyase activity
(e) a DNA encoding a polypeptide consisting of an amino acid sequence that has 1 to 5 amino acids deleted, substituted, inserted, or added in the amino acid sequence of SEQ ID NO: 3 or 4, and having chorismate-pyruvate lyase activity

2. The transformant according to claim 1, wherein the *Hydrogenophilus* bacterium is *Hydrogenophilus thermoluteolus.*

3. A method for producing 4-hydroxybenzoic acid, comprising a step of culturing the transformant according to claim 1 or 2 using substantially only carbon dioxide as a carbon source.
